# FASCICULE DE BREVET EUROPEEN

(11) **EP 2 016 968 B1**
(45) Date de publication et mention de la délivrance du brevet: **02.11.2011**
(21) Numéro de dépôt: 08159878.1
(22) Date de dépôt: 08.07.2008
(51) Int. Cl.: A61M 16/00, G05D 16/20

(54) **Dispositif d'aspiration de gaz d'anesthésie**
Ansaugvorrichtung für Anästhesiegas
Anaesthetic gas suction device

(30) Priorité: 16.07.2007 FR 0705133
(43) Date de publication de la demande: 21.01.2009
(73) Titulaire: Mil'S, 69740 Genas (FR)
(72) Inventeur: Gentien, M. Dominique, 69004 Lyon (FR)
(74) Mandataire: Maureau, Philippe

(56) Documents cités:
- WO-A1-80/01044
- WO-A1-2004/060459
- WO-A2-2006/124578
- DE-A1- 19 960 704
- US-A- 4 987 894

## Description

L'invention concerne un dispositif d'aspiration de gaz d'anesthésie.

Les gaz d'anesthésie sont utilisés dans le domaine médical, et en particulier en milieu hospitalier, afin d'anesthésier des patients pendant une opération. La gestion de l'anesthésie est assurée par des machines permettant notamment de régler le débit et la composition de gaz d'anesthésie, et de récupérer les gaz d'anesthésie expirés par le patient.

Afin d'éviter que ces gaz soient rejetés dans l'enceinte du bloc opératoire, ce qui pourrait avoir un effet néfaste sur le personnel médical, les gaz récupérés par la machine d'anesthésie sont injectés dans une conduite d'aspiration permettant d'évacuer ces gaz vers l'extérieur.

Un dispositif d'aspiration de gaz d'anesthésie comporte généralement au moins une prise qui, destinée au raccordement d'une machine d'anesthésie, est disposée à une extrémité d'une conduite d'aspiration, équipée de moyens d'aspiration.

Selon une première forme de réalisation connue, chaque prise est équipée d'un système venturi dans lequel un flux d'air médical est véhiculé afin de réaliser l'aspiration des gaz d'anesthésie. Cette première forme de réalisation nécessite une grande consommation d'air médical et oblige à stocker l'air utilisé. En outre, il est nécessaire de prévoir une conduite d'alimentation en air moteur et une conduite de refoulement pour le venturi dédiées à chaque prise.

Selon une seconde forme de réalisation connue, l'aspiration est centralisée par l'intermédiaire d'une conduite d'aspiration commune, équipée d'une turbine et d'une soupape casse-vide.

La soupape casse-vide permet de réguler la pression à l'intérieur de la conduite. La soupape casse-vide comporte un piston soumis à l'action d'un ressort taré en fonction des besoins, le mouvement du piston à l'encontre de la force du ressort permettant de réguler la pression des gaz dans la conduite.

Ce type de régulation a ses limites car les caractéristiques mécaniques de la soupape casse-vide varient au cours du temps, en fonction de paramètres extérieurs tels que la température ou le taux d'hygrométrie des gaz, ou en cas d'encrassement.

Une telle régulation ne permet pas non plus de réguler le débit d'aspiration lorsque le nombre de prises raccordées à une machine d'anesthésie varie.

Le document US 4,987,894 divulgue les caractéristiques du préambule de la revendication 1.

L'invention vise à résoudre ces inconvénients en proposant un dispositif d'aspiration de gaz d'anesthésie permettant une régulation fiable du débit d'aspiration des gaz d'anesthésie.

A cet effet, l'invention concerne un dispositif d'aspiration de gaz d'anesthésie, comportant au moins une prise qui, destinée au raccordement d'une machine d'anesthésie, est disposée à une extrémité d'une conduite d'aspiration, équipée de moyens d'aspiration tels qu'au moins une turbine ou une pompe, **caractérisé en ce que** d'une part, la conduite est équipée de moyens asservis de régulation de la pression du gaz circulant dans celle-ci, et en ce que, d'autre part, la prise comporte des moyens de réglage de la section de la conduite qui lui est associée.

Le débit des gaz à l'intérieur de la conduite étant dépendant de la pression à l'intérieur de celle-ci et de sa section, le réglage de la pression et de la section permettent de régler le débit. Il est ainsi possible d'obtenir un débit constant au niveau des prises, et ce, quel que soit le nombre de prises connectées à une machine d'anesthésie. En outre, le fait que la prise soit équipée de moyens de réglage de la section facilite le réglage du débit, puisque la prise est directement accessible à un opérateur. Enfin, la régulation asservie de la pression permet un ajustement automatique du débit à l'intérieur de la conduite, notamment en cas de modification de la section de la conduite par l'opérateur.

Selon une forme de réalisation de l'invention, les moyens asservis de régulation de la pression du gaz comportent une vanne de régulation de pression asservie à partir d'informations issues d'un capteur de pression du gaz circulant dans la conduite.

Selon une caractéristique de l'invention, les moyens de réglage de la section de la conduite comportent une vis de réglage apte à obstruer, au moins en partie, la conduite au niveau de la prise.

La vis de réglage peut ainsi être manipulée facilement par un opérateur. La position de la vis permet de modifier la section de la conduite au niveau de la prise. Inversement, il est possible de quantifier la section de la conduite en fonction de la position de la vis.

Préférentiellement, la prise est équipée de moyens d'immobilisation de la vis de réglage.

Les moyens d'immobilisation permettent d'éviter toute modification involontaire ou accidentelle du réglage de la section de la conduite.

Selon une caractéristique de l'invention, les moyens d'immobilisation comportent une vis de pression s'étendant sensiblement perpendiculairement à la vis de réglage et apte à venir prendre appui sur la surface latérale de celle-ci.

Avantageusement, les moyens d'immobilisation comportent un contre-écrou monté sur la vis de réglage.

Selon une forme de réalisation de l'invention, la prise est équipée d'une butée pour la vis de réglage, la butée délimitant une position d'obturation maximale.

La position d'obturation maximale peut ainsi être réglée pour assurer un débit de fuite minimal.

Préférentiellement, la conduite est équipée d'une pluralité de turbines ou pompes montées en parallèle.

La pluralité de turbines assure une aspiration suffisante lorsque de nombreuses prises sont raccordées à des machines d'anesthésie. Cette caractéristique permet également de pouvoir pallier une éventuelle panne d'une turbine.

Selon une caractéristique de l'invention, la conduite comporte une pluralité de prises disposées en parallèle et reliées à une conduite commune.

Chaque bloc opératoire peut ainsi comporter une ou plusieurs prises, l'ensemble faisant partie d'un réseau d'aspiration commun.

De toute façon, l'invention sera bien comprise à l'aide de la description qui suit, en référence au dessin schématique annexé représentant, à titre d'exemples, plusieurs formes de réalisation de ce dispositif d'aspiration de gaz d'anesthésie.
Figure 1 est une vue schématique d'une première forme de réalisation de l'invention ;
Figure 2 est une vue correspondant à la figure 1, d'une seconde forme de réalisation de l'invention ;
Figures 3 et 4 sont des vues en coupe d'une prise, de dessus, respectivement dans une première et une seconde positions de la vis de réglage;
Figure 5 est une vue en coupe, de face, de la prise ;
Figure 6 et 7 sont des vues correspondant respectivement aux figures 3 et 4, d'une variante de réalisation de la prise.

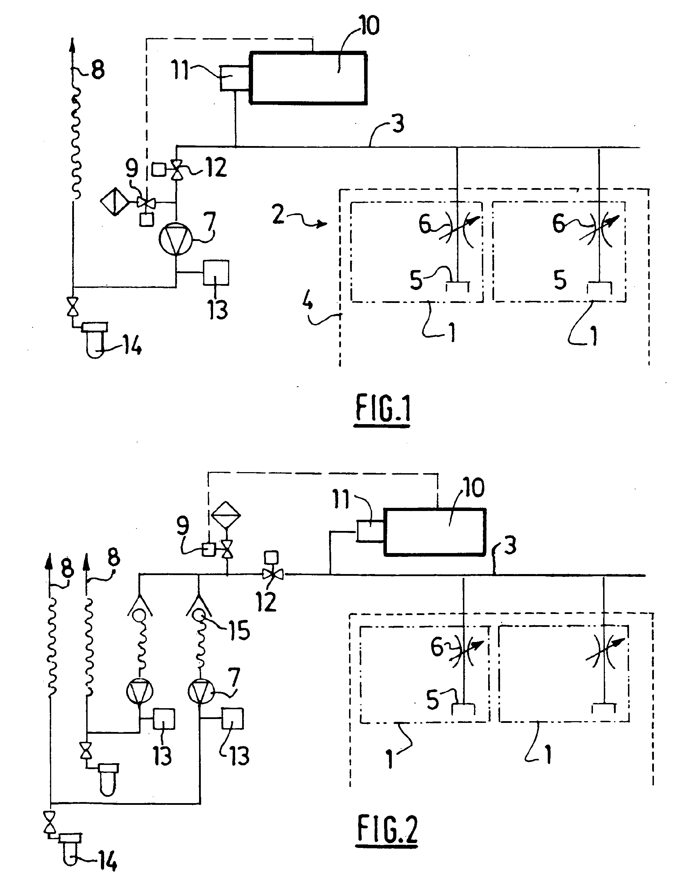

La figure 1 représente une première forme de réalisation d'un dispositif d'aspiration de gaz d'anesthésie selon l'invention.

Le dispositif d'aspiration comporte une pluralité de prises 1 qui, destinées au raccordement d'une machine d'anesthésie non représentée, sont disposées à une extrémité d'aspiration 2 d'une conduite d'aspiration 3. Les prises 1 sont disposées en parallèles les unes par rapport aux autres, les prises étant disposées dans l'enceinte d'au moins un bloc opératoire 4.

Chaque prise 1 est équipée d'un raccord 5 destiné au raccordement d'une machine d'anesthésie et de moyens de régulation de la section 6, dont la structure est détaillée ci-après.

La conduite 3 est équipée de moyens d'aspiration 7 tels qu'au moins une turbine ou une pompe, de manière à faire circuler les gaz des prises vers une extrémité de refoulement 8 située à l'extérieur.

La conduite 3 est en outre équipée, en amont des moyens d'aspiration 7, de moyens asservis de régulation de la pression du gaz circulant dans celle-ci.

Ceux-ci comportent une vanne 9 de régulation de pression asservie par un coffret de commande 10, délivrant un signal variant en fonction d'informations issues d'un capteur de pression 11 du gaz circulant dans la conduite 3.

La conduite 3 est en outre équipée d'une vanne d'isolement automatique ou d'un clapet à battant 12, d'un capteur de température 13 et d'un élément 14 apte à recueillir les produits d'une éventuelle condensation à l'intérieur de la conduite 3 afin d'éviter toute dégradation de la pompe ou de la turbine 7. Une telle condensation peut intervenir du fait que la conduite 3 débouche généralement, au niveau de son extrémité de refoulement 8, à l'extérieur d'un bâtiment, la conduite 3 étant alors soumise à des différences de température entre l'extérieur et l'intérieur du bâtiment.

On retrouve successivement, dans le sens de l'écoulement des gaz : les prises 1, le capteur de pression 11, la vanne d'isolement automatique ou le clapet à battant 12, la vanne de régulation de pression asservie 9, le capteur de température 13 et l'élément 14 permettant de recueillir la condensation.

Selon une seconde forme de réalisation du dispositif d'aspiration, ce dernier comporte une pluralité de turbines ou pompes 7 montées en parallèle les unes par rapport aux autres, un clapet anti-retour 15 étant disposé directement en amont de chaque turbine ou pompe 7.

L'activation d'une ou plusieurs turbines 7 dépend notamment du nombre de prises 1 actives, c'est-à-dire connectées à une machine d'anesthésie. L'activation des turbines 7 peut être commandée, en fonction des besoins, soit automatiquement par l'intermédiaire de moyens de commande appropriés, soit à la demande d'un opérateur.

Dans cette forme de réalisation, la conduite 3 comporte en outre une extrémité de refoulement 8 dédiée à chacune des pompes ou turbines 7. De manière alternative, ces dernières pourraient être reliées à une extrémité de refoulement commune.

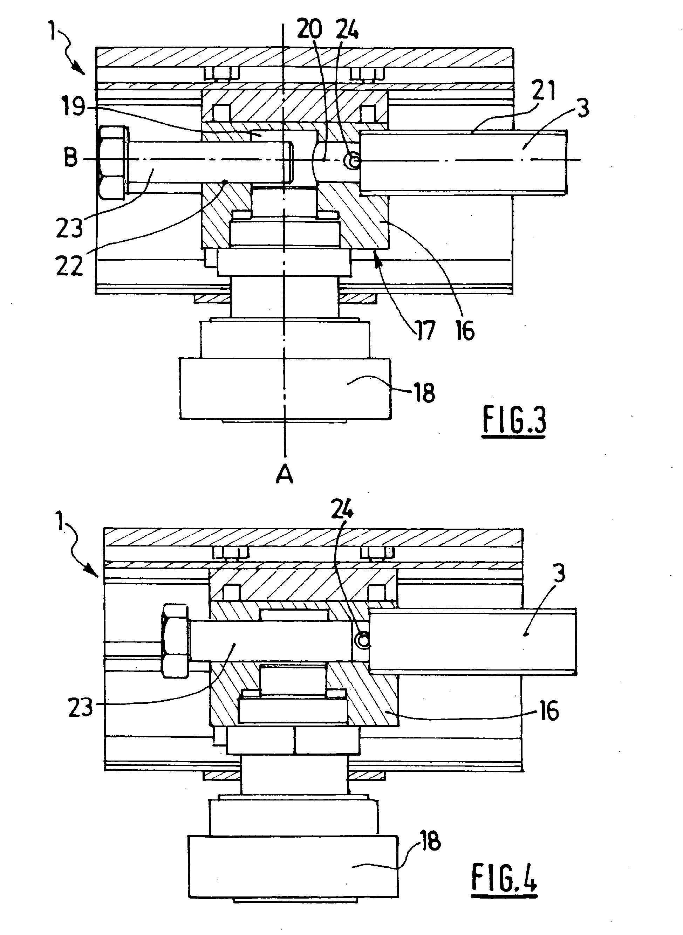

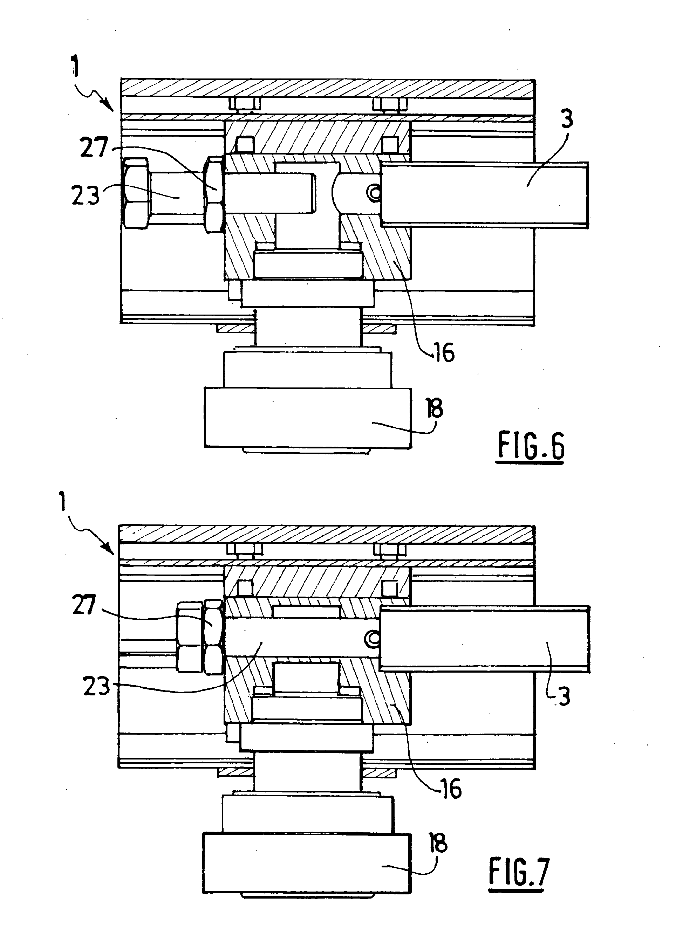

La structure de la prise 1 est représentée plus particulièrement aux figures 3 à 7, une première forme de réalisation étant représentée aux figures 3 à 5, une seconde forme de réalisation étant représentée aux figures 6 et 7.

La prise 1 comporte un corps 16 présentant une face avant 17 équipée d'un raccord normalisé 18 destiné au raccordement de la machine d'anesthésie.

Le corps 16 comporte une partie creuse délimitant une chambre interne 19 de passage des gaz, dans laquelle débouche le raccord 18 précité et à partir de laquelle s'étendant un trou d'échappement 20 formant une partie de la conduite d'aspiration 3 ou d'évacuation des gaz. L'axe A du raccord 18 et l'axe B du trou d'échappement 20 forment un angle de 90°. Le trou d'échappement 20 est en outre prolongé par un tronçon de conduite 21 monté sur le corps 16.

Le corps 16 comporte en outre un trou taraudé 22 qui, s'étendant suivant l'axe A, coopère avec une vis de réglage 23 apte à obstruer, au moins en partie, le trou d'échappement 20. La vis de réglage 23 forme ainsi en partie les moyens de réglage 6 représentés aux figures 1 et 2.

La vis 23 est ainsi déplaçable entre une première position représentée en figure 3 et une seconde position représentée en figure 4.

La prise est en outre équipée d'une butée de positionnement 24 faisant saillie à l'intérieur du trou d'échappement 20. Cette butée 24 permet de limiter la course de la vis de réglage 23 de sorte que, dans la position d'obstruction maximale représentée en figure 4, l'extrémité de la vis 23 soit en appui contre la butée 24.

Dans cette position, la conduite 3 n'est pas totalement obstruée puisque les gaz sont aptes à s'échapper entre les filets de la vis 23 et la paroi du trou d'échappement 20. La position de la butée 24 permet ainsi de régler un débit de fuite minimum.

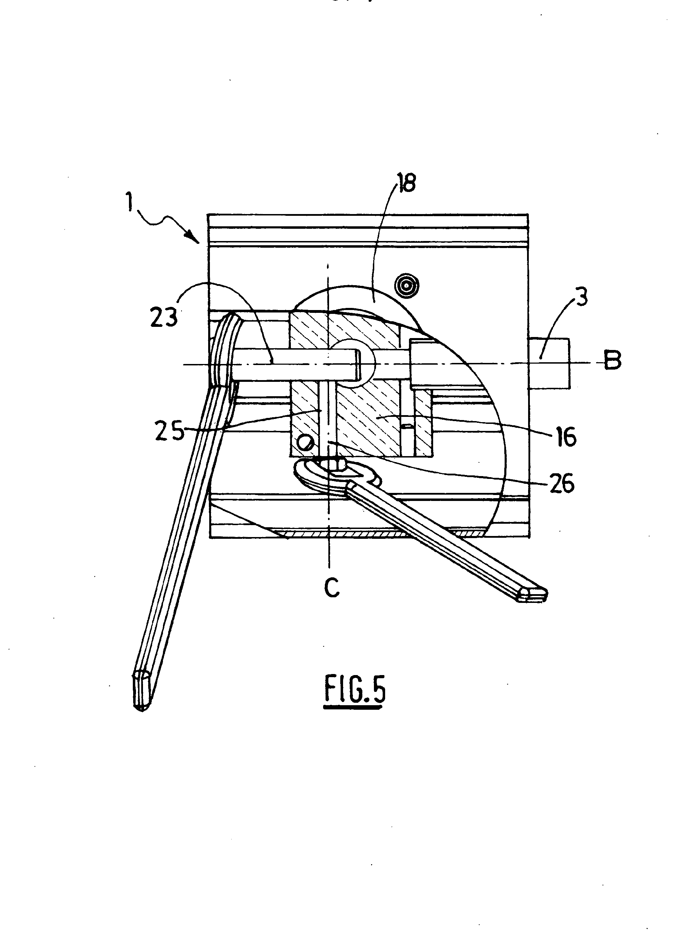

Comme cela est représenté en figure 5, le corps 16 de la prise 1 comporte en outre un trou taraudé additionnel 25 s'étendant selon un axe C sensiblement perpendiculaire à l'axe A du raccord et à l'axe B du trou taraudé 22.

Le trou taraudé additionnel 25 coopère avec une vis de pression 26 apte à venir en appui contre la surface externe de la vis de réglage 23, de manière à immobiliser cette dernière en position.

L'opérateur ajuste ainsi la position de la vis de réglage 23 en fonction de la section de passage choisie de la conduite 3 et serre ensuite la vis de pression 26.

Dans la première forme de réalisation représentée aux figures 3 à 5, la vis de réglage 23 et la vis de pression 26 sont toutes deux des vis à tête hexagonale. Dans une forme de réalisation alternative non représentée, les vis présentent des empreintes de type HC, c'est-à-dire à six pans creux. Tout type de tête ou d'empreinte peut naturellement être envisagé. Il est également possible de prévoir une empreinte particulière, correspondant à un outil spécifique de manière à ce que seul un opérateur en possession de l'outil spécifique puisse régler la section de la conduite au niveau de la prise.

Selon une seconde forme de réalisation représentée aux figures 6 et 7, la vis de pression 26 est remplacée par un contre-écrou 27 assurant la même fonction, à savoir l'immobilisation en position de la vis de réglage 23.

Comme il va de soi, l'invention ne se limite pas aux seules formes de réalisation de ce dispositif d'aspiration, décrites ci-dessus à titre d'exemple, mais elle embrasse au contraire toutes les variantes.

## Revendications

1. Dispositif d'aspiration de gaz d'anesthésie, comportant au moins une prise (1) qui, destinée au raccordement d'une machine d'anesthésie, est disposée à une extrémité (2) d'une conduite d'aspiration (3), équipée de moyens d'aspiration tels qu'au moins une turbine ou une pompe (7), **caractérisé en ce que** d'une part, la conduite (3) est équipée de moyens asservis de régulation de la pression (9, 10, 11) du gaz circulant dans celle-ci, et **en ce que**, d'autre part, la prise (1) comporte des moyens de réglage (23) de la section de la conduite (3) qui lui est associée.

2. Dispositif selon la revendication 1, **caractérisé en ce que** les moyens asservis de régulation de la pression du gaz comportent une vanne de régulation de pression (9) asservie à partir d'informations issues d'un capteur de pression (11) du gaz circulant dans la conduite (3).

3. Dispositif selon l'une des revendications 1 à 2, **caractérisé en ce que** les moyens de réglage de la section de la conduite (3) comportent une vis de réglage (23) apte à obstruer, au moins en partie, la conduite (20, 3) au niveau de la prise (1).

4. Dispositif selon la revendication 3, **caractérisé en ce que** la prise (1) est équipée de moyens d'immobilisation (26) de la vis de réglage (23).

5. Dispositif selon la revendication 4, **caractérisé en ce que** les moyens d'immobilisation comportent une vis de pression (26) s'étendant sensiblement perpendiculairement à la vis de réglage (23) et apte à venir prendre appui sur la surface latérale de celle-ci.

6. Dispositif selon la revendication 4, **caractérisé en ce que** les moyens d'immobilisation comportent un contre-écrou (27) monté sur la vis de réglage (23).

7. Dispositif selon l'une des revendications 3 à 6, **caractérisé en ce que** la prise (1) est équipée d'une butée (24) pour la vis de réglage (23), la butée (24) délimitant une position d'obturation maximale.

8. Dispositif selon l'une des revendications 1 à 7, **caractérisé en ce que** la conduite (3) est équipée d'une pluralité de turbines ou pompes (7) disposées en parallèle les unes par rapport aux autres.

9. Dispositif selon l'une des revendications 1 à 8, **caractérisé en ce que** la conduite comporte une pluralité de prises (1) disposées en parallèle et reliées à une conduite commune (3).

## Claims

1. A suction device for anesthesia gas, including at least one connector (1) that, intended to connect to an anesthesia machine, is arranged at one end (2) of a suction conduit (3), equipped with suction means such as at least one turbine or pump (7), **characterized in that** on the one hand, the conduit (3) is equipped with enslaved means (9, 10, 11) for regulating the pressure of the gas circulating therein, and **in that**, on the other hand, the connector (1) includes means (23) for adjusting the section of the conduit (3) associated therewith.

2. The device according to claim 1, **characterized in that** the enslaved means for regulating the pressure of the gas include a pressure regulating valve (9) enslaved from information coming from a pressure sensor (11) of the gas circulating in the conduit (3).

3. The device according to one of claims 1 to 2, **characterized in that** the means for adjusting the section of the conduit (3) include an adjusting screw (23) able to at least partially obstruct the conduit (20, 3) at the connector (1).

4. The device according to claim 3, **characterized in that** the connector (1) is equipped with means (26) for immobilizing the adjusting screw (23).

5. The device according to claim 4, **characterized in that** the immobilizing means include a pressure screw (26) extending substantially perpendicular to the adjusting screw (23) and able to bear on the side surface thereof.

6. The device according to claim 4, **characterized in that** the immobilizing means include a locknut (27) mounted on the adjusting screw (23).

7. The device according to one of claims 3 to 6, **characterized in that** the connector (1) is equipped with a stop (24) for the adjusting screw (23), the stop (24) defining a maximum closing position.

8. The device according to one of claims 1 to 7, **characterized in that** the conduit (3) is equipped with a plurality of turbines or pumps (7) arranged parallel to each other.

9. The device according to one of claims 1 to 8, **characterized in that** the conduit includes a plurality of connectors (1) arranged in parallel and connected to a shared conduit (3).

## Patentansprüche

1. Ansaugvorrichtung für Anästhesiegas, die mindestens ein Ansatzstück (1) aufweist, das, zur Verbindung an eine Anästhesiemaschine bestimmt, an einem Ende (2) einer Ansaugleitung (3) angeordnet ist, die mit Ansaugmitteln ausgestattet ist, wie beispielsweise mindestens einer Turbine oder einer Pumpe (7), **dadurch gekennzeichnet, dass** die Leitung (3) einerseits mit Steuerungsmitteln zur Druckregelung (9, 10, 11) des in ihr zirkulierenden Gases ausgestattet ist, und dass andererseits das Ansatzstück (1) Regelungsmittel (23) des Abschnitts der Leitung (3) aufweist, mit dem es verbunden ist.

2. Vorrichtung nach Anspruch 1, **dadurch gekennzeichnet, dass** die Steuerungsmittel zur Druckregelung des Gases ein Druckregelungsventil (9) aufweisen, das mit Informationen gesteuert wird, die von einem Drucksensors (11) des in der Leitung (3) zirkulierenden Gases ausgehen.

3. Vorrichtung nach einem der Ansprüche 1 bis 2, **dadurch gekennzeichnet, dass** die Regelungsmittel des Abschnitts der Leitung (3) eine Stellschraube (23) aufweisen, die imstande ist, die Leitung (20, 3) auf Ebene des Ansatzstücks (1) zumindest teilweise zu blockieren.

4. Vorrichtung nach Anspruch 3, **dadurch gekennzeichnet, dass** das Ansatzstück (1) mit Blockiermitteln (26) der Stellschraube (23) ausgestattet ist.

5. Vorrichtung nach Anspruch 4, **dadurch gekennzeichnet, dass** die Blockiermittel eine Druckschraube (26) aufweisen, die sich etwa senkrecht zur Stellschraube (23) erstreckt und imstande ist, sich auf der seitlichen Fläche derselben abzustützen.

6. Vorrichtung nach Anspruch 4, **dadurch gekennzeichnet, dass** die Blockiermittel eine Gegenmutter (27) aufweisen, die auf der Stellschraube (23) montiert ist.

7. Vorrichtung nach einem der Ansprüche 3 bis 6, **dadurch gekennzeichnet, dass** das Ansatzstück (1) mit einem Anschlag (24) für die Stellschraube (23) ausgestattet ist, wobei der Anschlag (24) eine maximale Verschlussstellung begrenzt.

8. Vorrichtung nach einem der Ansprüche 1 bis 7, **dadurch gekennzeichnet, dass** die Leitung (3) mit einer Vielzahl von Turbinen oder Pumpen (7) ausgestattet ist, die zueinander parallel angeordnet sind.

9. Vorrichtung nach einem der Ansprüche 1 bis 8, **dadurch gekennzeichnet, dass** die Leitung eine Vielzahl von Ansatzstücken (1) aufweist, die parallel angeordnet und mit einer gemeinsamen Leitung (3) verbunden sind.
